# EUROPEAN PATENT APPLICATION

(11) **EP 2 438 809 A1**
(43) Date of publication of application: **11.04.2012**
(21) Application number: 10782999.6
(22) Date of filing: 01.06.2010
(51) Int. Cl.: A01G 1/04, C12N 1/14

(54) **METHOD FOR THE LARGE-SCALE PRODUCTION OF MYCELIAL INOCULUM OF BASIDIOMYCETES**

(30) Priority: 02.06.2009 ES 200930246
(71) Applicant: Universidad Politécnica De Madrid, 28040 Madrid (ES)
(72) Inventor: JIMÉNEZ AGUILAR, María de la Consolación, E-28040 Madrid (ES); PÉREZ LECUONA, Ernesto, E-28040 Madrid (ES)
(74) Representative: Carvajal y Urquijo, Isabel
(86) International application number: PCT/ES2010/000241
(87) International publication number: WO 2010/139817

(57) **Abstract**

The present invention provides a method for obtaining fungal mycelium *in vitro* in pure culture isolated both from the fruiting bodies of fungal species of interest as well as from rootlet sections of plants which are in mycorrhizal association with said fungal species.

## Description

### Field of the Invention

The present invention is encompassed within the general field of agrobiotechnology and relates particularly to the large scale production of mycelial inoculum of fungal species.

### State of the Art

Up until now there are no references concerning the large scale production of mycelial inoculum of the fungal species *Amanita caesarea* and *Boletus aereus.* There is therefore a huge interest to find a simple, quick and economical method for obtaining mycelium of said species in pure culture for use in the nursery production of plants in mycorrhizal association from forest species of commercial interest for forest restoring and recovering mycological production in natural areas, as well as for the controlled nursery-plantation production of edible fungi of great commercial importance.

### Object of the Invention

The present invention provides a method for obtaining fungal mycelium *in vitro* in pure culture isolated both from the fruiting bodies of fungal species of interest as well as from rootlet sections of plants which are in mycorrhizal association with said fungal species.

Therefore, in a first aspect, the present invention relates to a method for obtaining fungal mycelium in pure culture which comprises the following steps:
a) isolating the fungal mycelium from fruiting bodies of fungi and/or rootlet sections of plants in mycorrhizal association,
b) inoculating the mycelium isolated in step a) in a solid modified oats culture medium (MOM),
c) purifying the mycelium,
d) growing the mycelium purified in step c) in a liquid modified BAF culture medium,
e) obtaining the mycelial inoculum

In a more particular aspect of the present invention, the fungal mycelium is isolated from fruiting bodies; in a more particular aspect, the fungus from which the mycelium is obtained is selected from the class Basidiomycetes, it is particularly selected from the genus *Amanita* and *Boletus.* In another more particular aspect, the fungus from which the mycelium is obtained is *Amanita caesarea.* In another particular aspect, the other fungus from which the mycelium is obtained is *Boletus aereus.*

In another more particular aspect of the present invention, the fungal mycelium is isolated from the rootlet sections of plants in mycorrhizal association, in a more particular aspect the mycelium is selected from the genus *Quercus* and *Cistus.*

In another more particular aspect of the present invention, the solid MOM culture medium of step b) contains calcium, nitrogen, phosphorus, potassium, magnesium, chlorine, sulfur, sucrose, yeast and agar as nutrients. In a more particular aspect, the solid modified oats culture medium of step b) contains 0.5 g of Ca (NO₃)₂ 4H₂O; 0.2 g of PO₄KH₂ ; 0.1 g of SO₄Mg 7H₂O; 0.1g of KCI; 5.0 g of sucrose; 3.5 g of oat powder; 0.1 g of yeast extract and 8.0 g of agar.

In another more particular aspect of the present invention, the purification of step c) is performed in a Petri dish (1) containing another smaller Petri dish (2) therein leaving a space between them (3) in which the MOM culture medium is deposited, which once the medium solidified the smaller Petri dish (2) is removed leaving a central space (4) where the mycelium inoculum is deposited.

In a more particular aspect of the present invention the modified BAF medium of step d) contains calcium, phosphorus, potassium, magnesium, manganese, zinc, iron, chlorine, sulfur, peptone, thiamine, biotin, inositol, folic acid, glucose and yeast as nutrients. In a more particular aspect, the modified BAF medium of the d) contains 0.1 g of Cl₂Ca; 0.5 g of PO₄KH₂; 0.5 g of SO₄Mg 7H₂O; 0.005 g of SO₄Mn; 0.001 g of SO₄Zn; 0.01 g of Cl₃Fe 7H₂O; 2.0 g of peptone; 0.0005 g of thiamine; 0.00001 g of biotin; 0.05 g of inositol; 0.0001 g of folic acid; 5.0-20.0 g of glucose and 0.2 g of yeast extract. In a more particular aspect, the modified BAF medium of step d) has a pH comprised between 5-6.5.

### Description of the Drawings

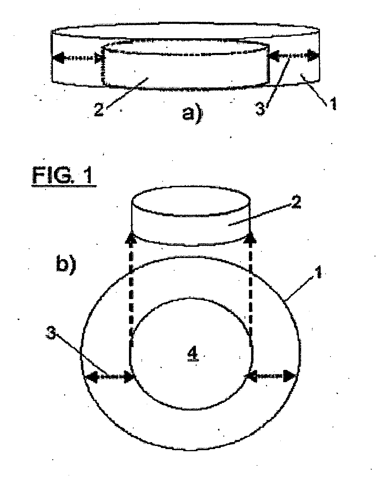

Figure 1 shows two different perspective views 1a and 1b of the system used for purifying the fungal mycelium.
Figure 1a corresponds to a Petri dish having a diameter of 9 cm (1) at the bottom of which a dish (2) having 5 cm of diameter which is shown with intermittent line is centrally placed. A 2 cm wide circular space (3) in which the melted culture medium is deposited, is created as a result of this configuration between both dishes.
Figure 1b shows the central space (4) that remains when, once the medium is cooled, the interposed dish is removed. There is located in said empty central space the mycelium inoculum which grows until reaching the peripheral section (3) where it meets the medium for purification, it is thus being freed from the possible presence of bacteria which are retained in the central section of the dishes since they cannot grow due to the absence of nutrients.

### Detailed Description of the Invention

The present invention relates to a method for obtaining mycelium *in vitro* in pure culture isolated both from the fruiting bodies of fungal species as well as from rootlet sections of cork oak plant (*Quercus suber*) and gum rockrose plant (*Cistus ladanifer*) which are in mycorrhizal association with fungi.

Fungal species of commercial interest in the present invention generally refers to fungal species of Basidiomycetes such as *Clytocybe sp.* or *Macrolepiota sp.,* and particularly to *Amanita caesarea* and *Boletus aereus.*

The process is started by individually isolating small sections from the innermost part of the hymenium of the mature carpophore pileus of *Amanita caesarea* and *Boletus aereus* from which the pellis has been previously removed in a laminar flow chamber. Then said sections were inoculated in maximum asepsis conditions in a laminar flow chamber, in plastic Petri dishes 5 cm in diameter previously sterilized in autoclave, to which the nutrient medium to be used was previously added: solid modified oats culture medium (MOM) the composition/liter of medium of which was: 0.5 g of Ca(NO₃)₂ 4H₂O; 0.2 g of PO₄KH₂ ; 0.1 g of SO₄Mg 7H₂O; 0.1g of KCI; 5.0 g of sucrose; 3.5 g of oat powder; 0.1 g of yeast extract and 8.0 g of agar. These dishes were kept in a chamber at 20°C until the mycelium grew occupying the entire dish surface.

The process described above was also performed using as inoculum rootlet sections isolated from the specimens of the mentioned plant species which had been previously put into mycorrhizal association with *Amanita caesarea* and *Boletus aereus,* and in which by means of optical microscopy examination the existence of mycorrhizal association with said fungi had been confirmed.

The mycelium obtained in both cases was then subjected to a purification process in order to eliminate the possibility of the existence of bacterial contamination, Petri dishes 9 cm in diameter being used to that end in which the culture medium (the MOM medium described above) was placed only in a 2 cm peripheral circular section (dishes prepared with the aid of dishes 5 cm in diameter such as shown in Figure 1). 1 x 1 cm sections of the solid MOM growth medium containing the fungal mycelium were placed in the central part of these dishes. These dishes were kept in a chamber at 20°C until the mycelium grew occupying the entire dish surface.

Once the mycelium is purified a small portion thereof was transferred for large scale growth to Erlenmeyer flasks containing liquid modified BAF culture medium with a composition/liter of medium: 0.1 g of Cl₂Ca; 0.5 g of PO₄KH₂; 0.5 g of SO₄Mg 7H₂O; 0.005 g of SO₄Mn; 0.001 g of SO₄Zn; 0.01 g of Cl₃Fe 7H₂O; 2.0 g of peptone; 0.0005 g of thiamine; 0.00001 g of biotin; 0.05 g of inositol; 0.0001 g of folic acid; 5.0-20.0 g of glucose, and, 0.2 g of yeast extract, the culture was kept under controlled conditions: 25°C, dark, pH 6 and constant stirring at 120 rpm.

A massive growth (1:10⁶) of the mycelium in pure culture prepared for inoculating the plants to be put into mycorrhizal association was obtained in 5 days.

This process is susceptible to be easily implemented in industrial type bioreactors for a quick production of large amounts of purified mycelial inoculum in controlled conditions.

## Claims

1. A method for obtaining fungal mycelium *in vitro* in pure culture which comprises the following steps:
a) isolating the fungal mycelium,
b) inoculating the mycelium isolated in step a) in a solid modified oats culture medium (MOM),
c) purifying the mycelium,
d) growing the mycelium purified in step c) in a modified biotin-aneurin-folic acid (BAF) culture medium,
e) obtaining the fungal mycelium.

2. The method for obtaining fungal mycelium according to claim 1, **characterized in that** said fungal mycelium is isolated from fruiting bodies of fungi.

3. The method for obtaining fungal mycelium according to claim 2, **characterized in that** the fungus from which said mycelium is obtained is selected from the genera *Boletus* and *Amanita.*

4. The method for obtaining fungal mycelium according to claim 3, **characterized in that** the fungus from which said mycelium is obtained is *Amanita caesarea.*

5. The method for obtaining fungal mycelium according to claim 3, **characterized in that** the fungus from which said mycelium is obtained is *Boletus aereus.*

6. The method for obtaining fungal mycelium according to claim 1, **characterized in that** said fungal mycelium is isolated from rootlet sections of plants in mycorrhizal association.

7. The method for obtaining fungal mycelium according to claim 6, **characterized in that** said plants from which the mycelium is obtained are selected from the genera *Quercus* or *Cistus.*

8. The method for obtaining fungal mycelium according to anyone of the preceding claims, **characterized in that** said solid modified oats culture medium (MOM) of step b) contains calcium, nitrogen, phosphorus, potassium, magnesium, chlorine, sulfur, sucrose, yeast and agar as nutrients.

9. The method for obtaining fungal mycelium according to claim 8, **characterized in that** said solid modified oats culture medium of step b) contains between 0.5 g of Ca(NO₃)₂4H₂O; 0.2 g of PO₄KH₂ ; 0.1 g of SO₄Mg 7H₂O; 0.1g of KCl; 5.0 g of sucrose; 3.5 g of oat powder; 0.1 g of yeast extract and 8.0 g of agar.

10. The method for obtaining fungal mycelium according to anyone of the preceding claims, **characterized in that** the purification of step c) is performed in a Petri dish containing another smaller Petri dish therein leaving a space between them where the MOM culture medium is deposited, which smaller Petri dish is removed once the medium is solidified leaving a circular space wherein the mycelium inoculum is deposited.

11. The method for obtaining fungal mycelium according to anyone of the preceding claims, **characterized in that** said liquid modified BAF medium of step d) contains calcium, phosphorus, potassium, magnesium, manganese, zinc, iron, chlorine, sulfur, peptone, thiamine, biotin, inositol, folic acid, glucose and yeast as nutrients.

12. The method for obtaining fungal mycelium according to anyone of the preceding claims, **characterized in that** said modified BAF medium of step d) contains 0.1 g of Cl₂Ca; 0.5 g of PO₄KH₂; 0.5 g of SO₄Mg 7H₂O; 0.005 g of SO₄Mn; 0.001 g of SO₄Zn; 0.01 g of Cl₃Fe 7H₂O; 2.0 g of peptone; 0.0005 g of thiamine; 0.00001 g of biotin; 0.05 g of inositol; 0.0001 g of folic acid; 5.0-20.0 g of glucose, and 0.2 g of yeast extract.

13. The method for obtaining fungal mycelium according to anyone of the preceding claims, **characterized in that** said liquid modified BAF medium of step d) has a pH comprised between 5-6.5.
